# EUROPEAN PATENT SPECIFICATION

(11) **EP 3 247 697 B1**
(45) Date of publication and mention of the grant of the patent: **08.01.2020**
(21) Application number: 16701340.8
(22) Date of filing: 22.01.2016
(51) Int. Cl.: C07C 209/16, C07C 209/52, C07C 211/42, C07C 251/20

(54) **PROCESS FOR THE RACEMIZATION OF ENANTIOMERICALLY ENRICHED 1-AMINOINDANE**
VERFAHREN ZUR RACEMISIERUNG VON ENANTIOMERENANGEREICHERTEM 1-AMINOINDAN
PROCÉDÉ POUR LA RACÉMISATION DE 1-AMINOINDANE ÉNANTIOMÉRIQUEMENT ENRICHI

(30) Priority: 22.01.2015 SI 201500008
(43) Date of publication of application: 29.11.2017
(73) Proprietor: Farma GRS, d.o.o., 8000 Novo mesto (SI)
(72) Inventor: RUZIC, Milos, 3000 Celje (SI); KRALJ, David, 8210 Trebnje (SI); KLOBCAR, Andrej, 8351 Straza (SI); PRUDIC, Darja, 8000 Novo mesto (SI); PLEVNIK, Miha, 1295 Ivancna Gorica (SI); PECAVAR, Anica, 8000 Novo mesto (SI); PLAPER, Igor, 8220 Smarjeske Toplice (SI); PUCELJ, Joze, 8000 Novo mesto (SI)
(74) Representative: Uexküll & Stolberg
(86) International application number: PCT/EP2016/051356
(87) International publication number: WO 2016/116607

(56) References cited:
- WO-A1-2012/116752
- US-A- 4 833 273
- GUOZHEN MA ET AL: "A Novel Synthesis of Rasagiline via a Chemoenzymatic Dynamic Kinetic Resolution", ORGANIC PROCESS RESEARCH AND DEVELOPMENT, vol. 18, no. 10, 17 October 2014 (2014-10-17), pages 1169-1174, XP055258423, US ISSN: 1083-6160, DOI: 10.1021/op500152g
- DATABASE REAXYS [Online] Elsevier Properties SA; XP002755712, Database accession no. 1073457 & JUSTUS LIEBIGS ANNALEN DER CHEMIE, vol. 679, 1964, pages 83-94,
- RODIONOV V N ET AL: "Selective reductive dimerization of homocubane series oximes", RUSSIAN JOURNAL OF ORGANIC CHEMISTRY, NAUKA/INTERPERIODICA, MO, vol. 47, no. 11, 8 December 2011 (2011-12-08), pages 1695-1702, XP019988595, ISSN: 1608-3393, DOI: 10.1134/S1070428011110078
- DATABASE REAXYS [Online] Elsevier Properties SA; XP002755713, Database accession no. 161544 & COMPTES RENDUS HEBDOMADAIRES DES SEANCES DE L'ACADEMIE DES SCIENCES, vol. 170, 1920, page 937,
- DATABASE REAXYS [Online] Elsevier Properties SA; XP002755714, Database accession no. 7054815 & ANNALES DE CHIMIE, vol. 11, no. 2, 1934, pages 225-242,

## Description

### Field of the invention

The present invention relates to an improved process for the racemization of (S)-1-aminoindane. (S)-1-aminoindane is formed as a side product in the process of preparation of (R)-1-aminoindane by enantiomeric resolution of racemic 1-aminoindane. (R)-aminoindane is a valuable intermediate in the process of preparation of rasagiline. Furthermore, the present invention involves the use of N-(2,3-dihydro-1H-inden-1-yl)-2,3-dihydro-1H-inden-1-imine and both its enantiomeric forms, i.e. (R)-N-(2,3-dihydro-1H-inden-1-yl)-2,3-dihydro-1H-inden-1-imine and (S)-N-(2,3-dihydro-1H-inden-1-yl)-2,3-dihydro-1H-inden-1-imine, for the preparation of 1-aminoindane, rasagiline and in the process of racemization of (S)-1-aminoindane. Moreover, the present invention relates to a process for preparing rasagiline using racemization of (S)-1-aminoindane.

### Background of the invention

Rasagiline is the common name for (R)-N-(prop-2-ynyl)-2,3-dihydro-1H-inden-1-amine, a compound of formula (I):

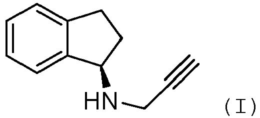

Several routes of synthesis have been reported for the preparation of rasagiline. The processes may be divided into two groups, namely (i) processes where racemic rasagiline is resolved in the last step of the synthesis, and (ii) processes where the chiral center is introduced already in an early stage of synthesis. Since half of the product is discarded when applying the enantiomeric resolution process in the last step of synthesis, it is desirable to include chiral centers at an earlier stage of the synthesis thus providing for a more economical synthesis and better yields. Even more, a more economical process is achieved when the unwanted enantiomer is racemized and re-used in the process of enantiomeric resolution.

Rasagiline was first disclosed in EP 436492 according to which it is prepared by reacting (R)-1-aminoindane and propargyl chloride. The document states that (R)-1-aminoindane may be prepared by the resolution of a racemic mixture of R- and S-enantiomers, e.g. by formation of diastereomeric salts with chiral acids.

The reaction of a racemic base with an optically active acid gives a pair of diastereomeric salts. Members of this pair exhibit different physicochemical properties (e.g. solubility, melting point, boiling point, adsorption, phase distribution) and can be separated due to these differences. The most common method for the separation of enantiomers is crystallization.

In the prior art several processes for the preparation of (R)-1-aminoindane from racemic 1-aminoindane using fractional crystallization of its diastereomeric salts are reported.

In Boll. Chim. Farm., 115, p. 489-500, (1976) a method using N-acetyl-L-leucine as a resolving agent in an aqueous solution is described. However, the R-isomer with desired enantiomeric purity was recovered from mother liquors only after 100 consecutive crystallizations; consequently also the yields were minimal.

In Biochemistry, 19, p. 2133-2139, (1980) a process using malic acid in absolute ethanol is disclosed. Four crystallizations from 96% ethanol have been required. Therefore, this process also suffers from a very low yield and from being time consuming.

US 4833273 discloses a process for the resolution of 1-aminoindane using (R)-N-acetyl-3,4-dimethoxyphenylalanine as the resolving agent. The drawback of the process is that the unwanted diastereomeric salt (R,S) is precipitated first from the reaction mixture, and that the desired salt (R,R) is recovered only by first concentrating and cooling mother liquors whereby an even less pure product was produced. This is evident from the physical data, such as melting point and specific rotation, reported for the product of the first crop and the second crop which substantially differ from each other. In addition to that, the resolving agent used is very expensive and, therefore, is not desirable for use in a large scale process.

In WO 2009/147430 a process which uses 2,3,4,6-di-isopopylidene-2-keto-L-gulonic acid as a resolving agent is disclosed. The diasteromeric salt formation proceeds in methanol, and recrystallization takes place in a solvent mixture of methanol and water. Enantiomeric purities for the resolving processes exemplified in the document were about 96%. However, in processes for the preparation of pharmaceutically active ingredients enantiomeric purity of more than 98% is required; another drawback of this process is the relatively high price of the reagent.

In WO 2012/116752 resolution of 1-aminoindane using L(+)-aspartic acid, L(-)-malic acid and (2R, 3R)-tartaric acid in methanol is disclosed. In case of resolution with L(+)-aspartic acid and (2R, 3R)-tartaric acid, enantiomeric purities of less than 94% are reported. L(-)-malic acid is selected as preferred resolution agent providing a product with an optical purity of 99.75% (HPLC), but the yield was lower than in the case of the other two acids.

Other known methods include derivatization of 1-aminoindane prior to resolution, and are disadvantageous as several protection and deprotection steps are required during the process, which results in lower yields and more possibilities for formation of process impurities.

The above mentioned documents are silent about the possibility of racemization of the undesired (S)-1-aminoindane, except for WO 2012/116752 which discloses racemization of the S-isomer by using an alcoholate in an organic solvent, which is preferably dimethylsulfoxide. However, the process which includes the use of alcoholates at relatively high temperatures may result in formation of unwanted side products. Consequently, the process requires laborious purification steps.

Ma et al. (Org. Process Res. Dev., 18, 10, 1169-1174 (2014)) disclose a synthethic route for the preparation of rasagiline mesylate that comprises a step of enzymatic dynamic kinetic resolution. By using Candida antarctica lipase B (CALB) and a Pd catalyst the chiral intermediate (R)-2,3-dihydro-1-indanamine is prepared. The synthetic route comprises in total 7 steps and is reported to result in an overall rasagiline mesylate yield of 25%.

Thus, there still exists a need for an economical process of racemization of the undesired (S)-1-aminoindane in order to obtain optimal yields in the synthesis of rasagilin.

### Summary of the invention

The present invention relates to a process for racemization of (S)-1-aminoindane or a mixture of (R)- and (S)-enantiomers of 1-aminoindane which mixture is enriched in (S)-1-aminoindane.

The processes of the present invention can be outlined in the following scheme:

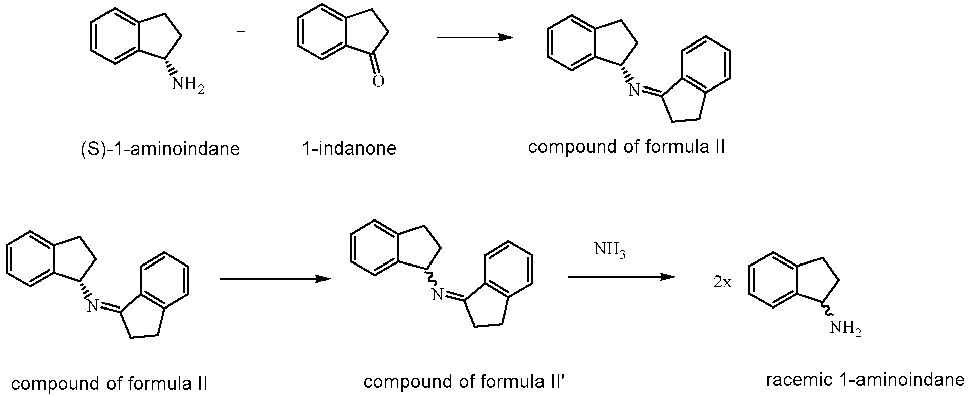

The racemization process according to the present invention can provide a yield of more than 50% of racemic 1-aminoindane, preferably of at least about 80% of racemic 1-aminoindane, and up to 100% of racemic 1-aminoindane, in relation to the starting (S)-1-aminoindane. As in the synthesis of rasagilin only (R)-1-aminoindane is used, the racemization of (S)-1-aminoindane according to the process of the present invention allows for a more than 50%, and preferably more than 80% more efficient synthesis of rasagilin, compared to a synthesis of rasagiline wherein (S)-1-aminoindane is not recycled.

A process where the unwanted (S)-isomer is recycled in the process of the synthesis of rasagiline is shown in the following scheme:

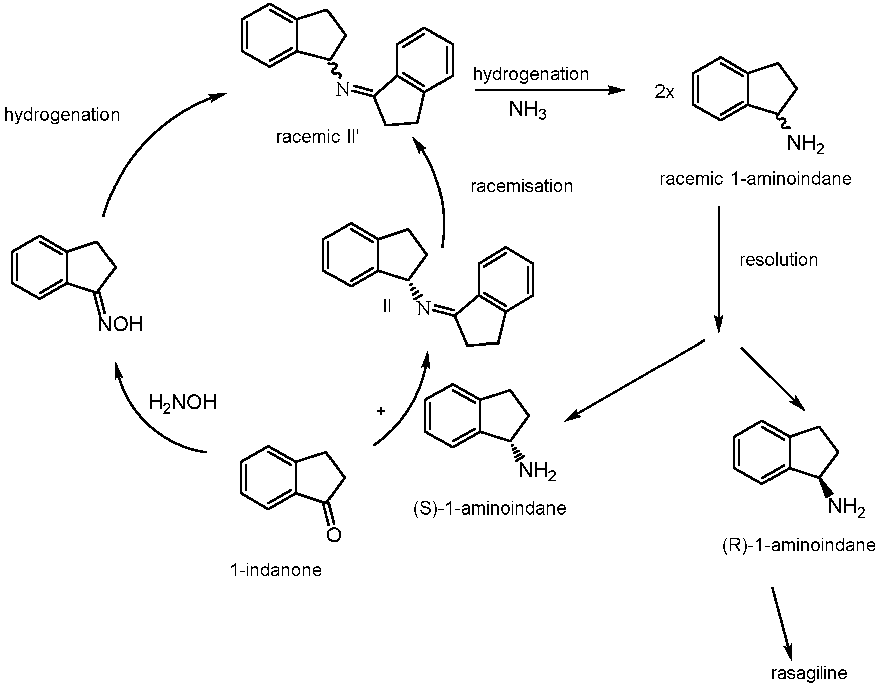

An efficient process for the preparation 1-aminoindane is disclosed in US 4833273. The process comprises the steps of converting the starting material 1-indanone to oxime and the hydrogenation of the oxime to obtain racemic 1-aminoindane. It has surprisingly been found out by the present inventors that the hydrogenation reaction proceeds via the formation of the symetrical Schiff base of formula II or II'. Confronted with the problem of developing an economical synthesis of rasagiline the present inventors have surprisingly found out that the unwanted (S)-1-aminoindane can be converted to the same intermediate, i.e. the Schiff base of formula II or II', by reaction with 1-indanone, which is also used as a starting material in the process of preparation of rasagiline. By the racemization process of the present invention the same starting material may be used and the same intermediate is formed as in the process where 1-aminoindane is prepared via oxime which allows that the same hydrogenation process can be used, and the product formed is of the same quality in terms of the impurity profile, consequently also the purification and the optical resolution process applied may be the same. The process of the present invention provides a very efficient process for the preparation of (R)-1-aminoindane, with yields above 50%, preferably above 80% and less waste material.

Furthermore, the use of the compound of formula II for the preparation of 1-aminoindane, the preparation of rasagiline, and in the process of racemization of (S)-1-aminoindane is disclosed. The chemical name of the compound of formula II 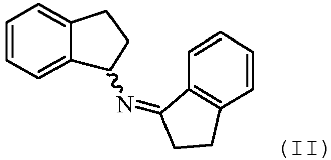 is N-(2,3-dihydro-1H-inden-1-yl)-2,3-dihydro-1H-inden-1-imine.

The present invention also specifically relates to the use of both enantiomers of compound II, namely IIa and IIb, which are represented by the structural formulas below:

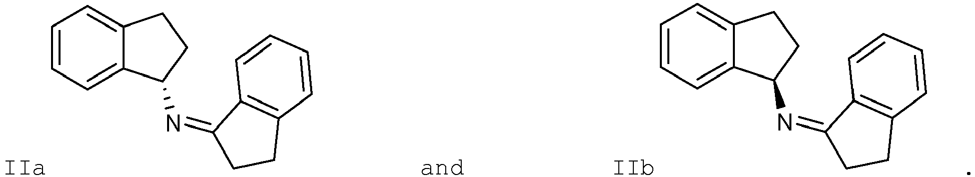

The chemical name of the compound of formula IIa is (R)-N-(2,3-dihydro-1H-inden-1-yl)-2,3-dihydro-1H-inden-1-imine, and the chemical name of compound IIb is (S)-N-(2,3-dihydro-1H-inden-1-yl)-2,3-dihydro-1H-inden-1-imine.

In addition, the present invention is directed to a process for preparing rasagiline which includes the step of racemization of (S)-1-aminoindane according to the invention.

### Detailed description of the invention

In a first aspect, the present invention relates to a process for racemization of (S)-1-aminoindane or a mixture of (R)- and (S)-enantiomers of 1-aminoindane which mixture is enriched in (S)-1-aminoindane, wherein the process results in the formation of racemic or nearly racemic 1-aminoindane.

(S)-1-aminoindane or a mixture of (R)- and (S)- enantiomers of 1-aminoindane, enriched in (S)-1-aminoindane, may be obtained as a side product of any resolution method of 1-aminoindane, such as the resolution via diasteromeric salt formation, resolution via diasteromeric complex formation, resolution via diastereomeric compound formation and/or resolution via chromatography. Preferably, (S)-1-aminoindane or a mixture of (R)- and (S)-enantiomers of 1-aminoindane, enriched in (S)-1-aminoindane, recovered from the mother liquor after separation of diastereomeric salts of 1-aminoindane with an optically active acid is used. According to the present invention, (S)-1-aminoindane is racemized by reaction with 1-indanone, the latter also being used as a starting material in the process of preparation of racemic 1-aminoindane.

As used herein, the term "racemic 1-aminoindane" refers to a mixture of (R)-1-aminoindane and (S)-1-aminoindane, having an S/R ratio of about 50/50. The term "nearly racemic 1-aminoindane" refers to a mixture of (R)-1-aminoindane and (S)-1-aminoindane, having an S/R ratio ranging from about 55/45 to about 45/55. Preferably, the nearly racemic 1-aminoindane has an S/R ratio ranging from about 55/45 to about 45/55. More preferably, the nearly racemic 1-aminoindane has an S/R ratio ranging from about 52/48 to about 48/52. Even more preferably, the nearly racemic 1-aminoindane has an S/R ratio ranging from about 51/49 to about 49/51.

As used herein, the term "(S)-1-aminoindane enriched mixtures" refers to a mixture of (R)-1-aminoindane and (S)-1-aminoindane, having an S/R ratio higher than about 55/45, preferably higher than 65/35, more preferably higher than 70/30, and up to 100/0, where the term "(S)-aminoindane enriched mixtures" is substituted with the term "pure (S)-aminoindane".

As used herein, "racemization" of a "(S)-1-aminoindane enriched mixtures" means a process of converting the said mixture to a racemic 1-aminoindane, or less (S)-1-aminoindane enriched mixtures, i.e. a mixture comprising (S)-1-aminoindane in a lower amount compared to the starting mixture, wherein the starting mixture is a material comprising (S)-1-aminoindane such that the S/R ratio of the starting mixture is 55/45 or above about 65/35, preferably, above about 70/30, more preferably above about 80/20.

The starting mixture of (S)-1-aminoindane and (R)-1-aminoindane comprises more S-isomer, i.e. the starting mixture comprises the S-isomer in a higher amount than the R-isomer, and the "racemization" reduces the relative amount of (S)-1-aminoindane and increases the relative amount of (R)-1-aminoindane to form the racemic or nearly racemic 1-aminoindane.

According to one embodiment, the racemization processes of the present invention can be outlined in the following scheme:

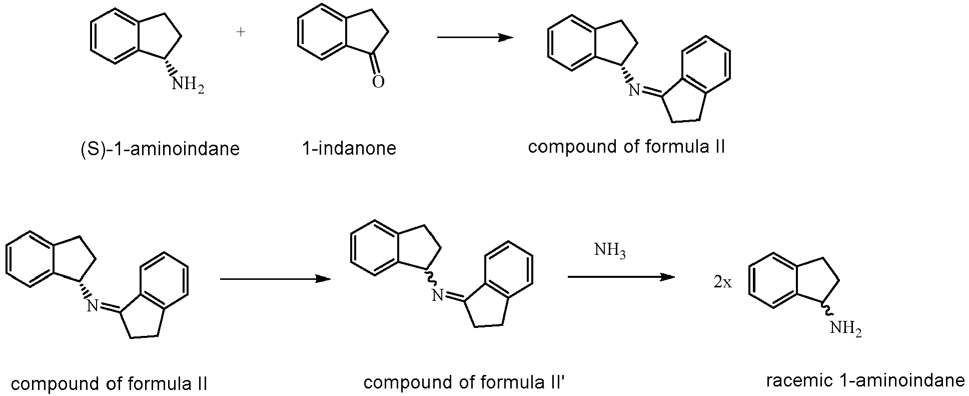

According to a preferred embodiment, the present invention provides a racemization process comprising the following steps:
(a) providing a material comprising (S)-1-aminoindane enriched mixtures;
(b) mixing the material with 1-indanone and at least one organic solvent in the presence of an aprotic base to form an optically active compound of formula II, 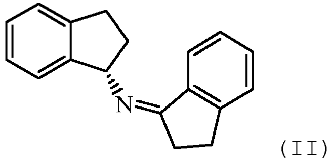
(c) racemization of compound of formula II by treatment with a base to get the compound of formula II',
(d) hydrogenating of the racemic compound of formula II', to obtain racemic 1-aminoindane.

In above steps (c) and (d), the terms "compound of formula II'" and "racemic compound of formula II'" refer to the compound of Formula II' shown in the scheme immediately above. Although depicted as optically pure S-enantiomer, the optically active compound of formula II obtained in step (b) may also comprise the corresponding R-enantiomer, depending on the amount of the R-enantiomer in the starting mixture. Generally, the S/R ratio of the compound II obtained in step (b) corresponds to, i.e. is essentially the same as, the S/R ratio of the starting material in step (a).

Optionally, the starting material comprising (S)-1-aminoindane used in step (a) further comprises wherein the amounts of (S)-1-aminoindane and (R)-1-aminoindane in the starting material are not identical and wherein the S/R ratio is greater than about 55/45 (w/w, or mole/mole).

Preferably, the starting material comprising (S)-1-aminoindane is obtained from a mother liquor comprising (S)- and (R)-1-aminoindane acid addition salts, wherein said addition salts are obtained from the optical resolution of racemic 1-aminoindane by using a chiral acid. The (R)- and (S)-1-aminoindane acid addition salts in the solution are preferably neutralized to obtain a mixture of (S)-1-aminoindane and (R)-1-aminoindane which is then used as the starting material in the process of the present invention. In case no neutralization of the 1-aminoindane acid addition salt is performed prior to the racemization of the material comprising (S)-1-aminoindane and/or its acid addition salt, the workup after the performed racemization may prove to be more laborious, e.g. more crystallizations may be required. Preferably, the (S)- and (R) -1-aminoindane acid addition salts are (S)-and (R)-1-aminoindane N-acetyl-L-glutamate or (S)-and (R)-1-aminoindane malate.

The organic solvent used in step (b) may be a non-polar solvent, such as toluene, xylene, tetrahydrofuran (THF), cyclohexane, hexane, 1,4-dioxane, or an alcohol, preferably an aliphatic alcohol, such as methanol, ethanol, propanol, isopropanol, butanol, sec-butanol, or a mixture thereof. Preferably the organic solvent is selected from toluene, xylene and methanol. The amount of the organic solvent used may be, for example, about 2 to 50 mL per gram, preferably about 5 to 20 mL per gram, and most preferably about 10 to 15 mL per gram, of the starting material comprising (S)-1-aminoindane.

The aprotic base used in step (b) may be selected from primary, secondary, or tertiary alkylamines, such as n-ethyldiisopropylamine, triethylamine, diethylamine, butylcyclohexylamine, diisopropylamine, dibutylamine, or heterocyclic amines, such as pyrrolidine, N-methylpyrrolidinone, N-methylpyrrolidine, piperidine, pyridine, or an alkyl substituted derivative thereof, such as dimethylamino pyridine (DMAP), or a mixture thereof or from inorganic bases or metal hydroxydes like KOH, NaOH or other alkali or alkali metal alcoholates, like potassium tert-butoxide or other alchololates. The mixture of various bases may be mixed at any ratio.

The amount of base may be of about 3 to about 5 molar equivalents with reference to the starting material comprising (S)-1-aminoindane. Preferably, the amount of the base is of 0.9 to 1.2 molar equivalents with reference to the starting material comprising (S)-1-aminoindane.

The molar ratio of 1-indanone used in step (b) to the amount of 1-aminoindane in the (S)-1-aminoindane enriched mixture preferably ranges from 0.5:1 to 1.5:1, more preferably from 0.6:1 to 1.2:1, most preferably from 0.7:1 to 0.8:1.

Typically, the reaction mixture is heated to a temperature of 40°C to the boiling point of the reaction mixture, preferably to a temperature of 50°C to 110°C, depending on the organic solvent used during the reaction.

The reactor is preferably equipped with a device which allows the removal of water formed as a by-product of the reaction, such as a Dean Stark apparatus.

The reaction time of step (b) is from 1 hour to 24 hours, preferably from 4 hours to 8 hours, most preferably 5 hours to 7 hours.

The racemization of step (c) is carried out by the addition of a strong base to the reaction mixture obtained in step (b). The base may be selected from alkali metal alcoholates, such as sodium or potassium methanolate, sodium or potassium ethanolate, sodium or potasium ethanolate, sodium or potassium t-butoxide, or tertiary amines such as 1,8-diazabicyclo[5.4.0]undec-7-ene (DBU), 1,5-diazabicyclo[4.3.0]non-5-ene (DBN), or hydroxides such as sodium or potassium hydroxide, or a mixture thereof.

The amount of the strong base to be used in step (c) is, in general, from 0.5 to 2 molar equivalents, preferably from 0.8 to 1.5 molar equivalent based on the optically active Schiff base of formula II'.

The reaction of step (c) is preferably carried out at a temperature of 40°C to boiling point of the reaction mixture, preferably to a temperature of 50°C to 120°C, depending on the organic solvent used during the reaction.

After the racemization is completed the reaction mixture is filtered to remove any solid unreacted particles, and the filtrate comprising the racemic Schiff base is used directly in step (d), without any further purification. The solvent which was used for the racemization step may be replaced with a more appropriate solvent for the hydrogenation step, if necessary.

In step (d) the racemised Schiff base of formula II is hydrogenated to produce 1-aminoindane. Preferably, hydrogenation is carried out in an organic solvent in the presence of a hydrogen source, a catalyst and ammonia.

The hydrogen source may preferably be gaseous hydrogen (H₂). The pressure of H₂ used may vary from 1 atmosphere to 5 atmospheres, preferably from 3 to 4 atmospheres.

The catalyst may be selected from metal catalysts such as platinum, palladium, rhodium, and ruthenium, catalysts based on nickel such as Raney nickel and Urushibara nickel. Preferably, Raney Ni is used as catalyst during the hydrogenation of Schiff base (II).

The organic solvent used in step (d) may be the same as the solvent used in steps (b) and (c). Preferably, step (b), step (c) and step (d) are performed in the same solvent, without isolation of the products obtained in steps (b) and (c). It was found out by the present invention that the presence of water in the reaction mixture results in the formation of unwanted by-products therefore the presence of water should be minimized. Preferably, the reaction is performed in the absence of water.

The temperature range during hydrogenation may vary from 10°C to 60°C, preferably from room temperature to 50°C, most preferably from 35 to 45°C. The reaction time of step c) is from 2 to 24 hours, preferably 16 to 24 hours.

After the reaction is finished the reaction mixture is cooled to about 20°C to 0°C, and the resulting racemic 1-aminoindane, or nearly racemic 1-aminoindane, in case the reaction was terminated early, is recovered. Preferably, the obtained racemic 1-aminoindane is recovered or isolated by a procedure known in the art, such as filtration, centrifugation, concentration and/or recrystallization. Preferably, the recovery comprises filtering of the catalyst, washing and concentrating the filtrate. Preferably, the washing of the filtrate is performed with the same organic solvent as used during the reaction process. Preferably, the obtained product is stored in inert atmosphere, at temperatures below 10°C, and protected from light. The obtained nearly racemic 1-aminoindane has a S/R ratio of (S)-1-aminoindane and (R)-1-aminoindane lower than 55/45. Preferably, the nearly racemic 1-aminoindane has an S/R ratio ranging from about 55/45 to about 45/55. More preferably, the nearly racemic 1-aminoindane has an S/R ratio ranging from about 52/48 to about 48/52. Even more preferably, the nearly racemic 1-aminoindane has an S/R ratio ranging from about 51/49 to about 49/5 Most preferably, the racemization reaction is carried out until a racemic mixture (50:50) of (S)-1-aminoindane and (R)-1-aminoindane is reached.

In one of the embodiments, the present invention also provides a neutralization method of an (S)-1-aminoindane acid addition salt for obtaining (S)-1-aminoindane, comprising the reaction of the material comprising an acid addition salt of (S)-1-aminoindane, with at least one base in an aqueous solvent mixture. The said material optionally further comprises an acid addition salt of (R)-1-aminoindane, and the molar ratio of the (S)-1-aminoindane acid addition salt and the (R)-1-aminoindane acid addition salt is higher than about 55:45. Accordingly, the process of the invention according to the first aspect is optionally characterized in that prior to step (a) a mixture comprising an acid addition salt of (S)-1-aminoindane and optionally an acid addition salt of (R)-1-aminoindane, wherein the amount of the acid addition salt of (S)-1-aminoindane is higher than the amount of the acid addition salt of is neutralized.

If the starting material comprises the acid addition salt of (S)-1-aminoindane and acid addition salt of (R)-1-aminoindane with the molar ratio of greater than about 55:45 , the product of the neutralization method of the present invention will also have a molar ratio of (S)-1-aminoindane to (R)-1-aminoindane greater than about 55:45 .

As used herein, "material" may be understood either as a solid mixture comprising (S)-1-aminoindane and or their acid addition salts, or as an aqueous or non-aqueous solution comprising (S)-1-aminoindane and or their acid addition salts, such as e.g. a mother liquid obtained by a fractional crystallization. The mother liquor is typically obtained from an optical resolution process of racemic 1-aminoindane, wherein the precipitated (R)-1-aminoindane acid addition salt is removed by filtration, and the remaining substance is used as the mother liquor in the neutralization step of the process of the present invention, wherein the mother liquor contains (S)- and (R)-1-aminoindane acid addition salts in a ratio of (S)-1-aminoindane and (R)-1-aminoindane above about 65/35 (w/w, or mole/mole). Preferably, the acid addition salt is formed with N-acetyl-L-glutamic acid or L-(-)-malic acid. Accordingly, as used herein, the term "material comprising (S)-1-aminoindane enriched mixtures" denotes any composition comprising (S)-1-aminoindane or a salt thereof and optionally (R)-1-aminoindane or a salt thereof, wherein the amount of (S)-1-aminoindane or a salt thereof is higher than the amount of (R)-1-aminoindane or a salt thereof.

The solvent mixture in which the neutralization of the acid addition salt(s) of (S)- or (R)-1-aminoindane is performed, may comprise water and a water immiscible solvent, preferably ethyl acetate. Optionally also a chelating agent, such as e.g. ethylenediaminetetraacetic acid (EDTA), may be added to the neutralization mixture.

The concentration of the material for the neutralization of the acid addition salt in the solvent mixture is preferably about 0.01 to about 0.3 g per mL of water, more preferably the concentration is about 0.05 to about 0.15 g per mL of water, and most preferably about 0.05 to about 0.1 g per mL of water. A preferred molar ratio of the at least one base to the material is about 0.9 to about 3.

Most preferably, the acid addition salt of aminoindane to be neutralized is a water soluble salt of an optically active acid, such as N-acetyl-L-glutamic acid. Additional examples of organic optically active acids are: 2,3,4,6-di-isopopylidene-2-keto -L-gulonic acid, L(+)-aspartic acid, L(-)-malic acid and (2R, 3R)-tartaric acid. If the acid addition salt to be neutralized is not water soluble, a slurry is obtained instead of a solution.

Preferably, the temperature during the neutralization reaction of at least one base with the material, is of about 5°C to about 60°C, more preferably of about 20°C to about 50°C, more preferably from about 20°C to about 40°C, and, most preferably, from 20°C to 25°C.

Preferably, the neutralization mixture is stirred for about 5 minutes to about 5 hours, more preferably, for about 15 minutes to about 2 hours, and most preferably, for about 30 minutes.

The base used for the neutralization of the acid addition salt of 1-aminoindane containing material may be selected from the group consisting of ammonia, alkaline earth metal hydroxide, alkali metal hydroxide, alkali metal carbonates, alkaline earth metal carbonates, alkali metal bicarbonates, alkaline earth metal bicarbonates and amines. Preferably, the alkali metal is selected from the group consisting of potassium and sodium. Preferably, the carbonate is selected from the group consisting of potassium carbonate and sodium carbonate. Preferably, the bicarbonate is sodium bicarbonate or potassium bicarbonate. The base may be added as a solid or an aqueous solution. Preferably, the base is added as an aqueous solution. Preferably, the aqueous solution of the base is added gradually. An ideal pH after addition of the base is about 7 to about 12, preferably about 10 to about 11.

After the neutralization is accomplished, the obtained mixture comprising (S)-1-aminoindane is isolated. Preferably, the isolation is carried out by extraction. The aqueous phase and the water immiscible solvent phase (s) are then separated, and the aqueous phase may be additionally washed with a water immiscible solvent. The organic phases are collected and concentrated to obtain the oily product. Preferably the neutralization reaction mixture is extracted with a water immiscible organic solvent, selected from the group of esters such as ethyl acetate, butyl acetate, aromatic hydrocarbons such as toluene, benzene, xylene, ethers such as diethyl ether, di-isopropyl ether, methyl-t-butyl ether, hydrocarbons such as heptane, hexane, pentane, cyclohexane, halogenated hydrocarbons such as chloroform, carbon tetrachloride, dichloromethane, 1,2-dichlorethane, preferably solvent mixture contains ethyl acetate.

The compound N-(2,3-dihydro-1H-inden-1-yl)-2,3-dihydro-1H-inden-1-imine (the compound of formula (II)), as well as both of its enantiomeric forms, i.e. (R)-N-(2,3-dihydro-1H-inden-1-yl)-2,3-dihydro-1H-inden-1-imine and (S)-N-(2,3-dihydro-1H-inden-1-yl)-2,3-dihydro-1H-inden-1-imine, and a racemic mixture of said enantiomers, can be used for the preparation of 1-aminoindane, rasagiline and in the process of racemization of (S)-1-aminoindane.

As described above, the compounds can be obtained as intermediates in the process according to the first aspect of the invention. It has been surprisingly found that the compound of formula (II) is a useful intermediate in the effective racemization of (S)-1-aminoindane enriched mixtures and, therefore, in the preparation of racemic 1-aminoindane and subsequently rasagiline.

In a second aspect, the present invention is directed to a process for preparing rasagiline which includes the step of racemization of (S)-1-aminoindane according to the first aspect of the invention. Accordingly, the present invention also relates to a process for preparing rasagiline or a pharmaceutically acceptable salt thereof by converting optically pure (R)-1-aminoindane into rasagiline or a pharmaceutically acceptable salt thereof, wherein the optically pure (R)-1-aminoindane is prepared from a racemate of 1-aminoindane prepared by a process according to the racemisation process of the present invention. The conversion of the optically pure (R)-1-aminoindane into rasagiline or a pharmaceutically acceptable salt thereof can be performed by processes known in the art such as processes described in EP 436492, EP 812190 ,WO 2009/14730 or WO 2012/116752. In order to obtain rasagiline, 1-aminoindane can be reacted with propargyl chloride or other reagents, such as other propargyl halides or propargyl sulphonates, such as propargyl benzensulphonate.

The present invention is further described in greater detail in the following examples.

### Experimental

### Methods

**The FT-IR spectra** were obtained with a FT-IR spectrometer Perkin Elmer Frontier at a resolution of 4 cm⁻¹ from 4000 cm⁻¹ to 400 cm⁻¹.

### NMR spectroscopy

Nuclear magnetic resonance (NMR) spectra were acquired using a Varian VNMRS 400 MHz spectrometer at 25°C. Samples were prepared as solutions in a suitable deuterated solvent.

### Chromatographic purity, enantiomeric purity and assay were determined by HPLC method

The product's purity was assessed by high pressure liquid chromatography (HPLC) using a octadecyl silica column (type YMC Triart C-18 150 x 4.6 mm i.d., 3 µm particles); column temperature: 25°C; detector: UV 210 nm; flow rate: 1.0 mL/min; injection volume: 2 µL; mobile phase A: 0.01M phosphate buffer solution pH 8; mobile phase B: 90% acetonitrile; Gradient: 0'=2%B, 10'=30%B, 29'-33'=100%B, 37'-42'=2%B. This HPLC method is used for the analysis of chromatographic purity and assay.

**Enantiomeric purity** was assessed by HPLC method using a polysaccharide based chiral column (type Chiralpak AS-H, 250 x 4.6 mm, 5 µm particles) . Mobile phase (which was a mixture of hexane, ethanol and butylamine in the ratio 980:17:0.4 (V/V/V); flow-rate: 0.6 mL/min; UV detector: 265 nm; injection volume: 15 µL) was used for separation.

### Sample preparation:

Assay and chromatographic purity: The sample solution was prepared in a concentration of about 1to abot- 1.5 mg/mL. Dilution solvent was acetonitrile and water.

Enantiomeric purity: The sample solution was prepared in concentration of about 10 mg/mL. Dilution solvent was a mixture of ethanol and 2-propanol.

### Calculation:

Chromatographic purity: Area percent method was used. Solvent peaks were disregarded.
Assay: External standard method was used.
Enantiomeric purity: Area percent method was used.

### Examples

### Example 1 (Preparation of starting material):

### Preparation of 1-indanone oxime

The reactor was charged with 200 g of 1-indanone (1.5133 mol) and 1440 mL of 96 % ethanol and stirred at room temperature (20° - 25°C) for 15 minutes. To the mixture 189.3 g of hydroxylamine hydrochloride (2.7239 mol) was added and stirred for 10 minutes. A thick suspension was formed to which 1040 mL of 2N NaOH was added, and the suspension was stirred at room temperature for 1 hour. The solvent was evaporated under reduced pressure to obtain a solid residue. To the residue 2400 mL of water, and 2500 mL of ethyl acetate was added and stirred for 15 minutes. The phases were separated and the aqueous phase was washed with 2500 mL of ethyl acetate. The phases were separated. Both organic phases were combined and evaporated under reduced pressure at temperatures below 40 ° C. 204 g of the title product was obtained in a molar yield of 91.6%.

### Example 2 (Preparation of starting material):

### Preparation of racemic 1-aminoindane

The reactor was charged at 25°C with 585.8 g 1-indanone oxime and 4960 mL of 4.2 M ammonia in methanol, after which 117 g of Raney Ni were added to the reactor in the form of a suspension in methanol. The mixture was hydrogenated under stirring at a constant pressure of 3.5 ± 0.1 bar. The hydrogenation reaction was carried out at a temperature of 42 ± 2°C for 20 hours. After completion of the reaction, the reaction mixture was cooled to 25 ± 2°C and the suspension was filtered to separate the catalyst. The filtrate was concentrated under reduced pressure at a temperature of less than 40 °C to constant weight. 558.0 g of an oily product were obtained (assay: 95.1% HPLC, molar yield 100%). The product was stored in an inert atmosphere at a temperature below 10°C and protected from light.

### Example 3

### Preparation of diastereomeric salt (R, S)-1-aminoindane N-acetyl-L-glutaminate

42.9 g of (R, S)-1-aminoindane, 42.9 mL of ethanol and 174 mL of TBME were added to the reactor. To this mixture 42.6 g of N-acetyl-L-glutamic acid was added under stirring at room temperature. The ratio of (R,S)-1-aminoindane and N-acetyl-L-glutamic acid was 1/0.7. After a few minutes, the product started to crystallize from the solution and a thick suspension was formed, to which 87 mL of TBME were added to obtain a miscible suspension, which was further stirred for 30 minutes at room temperature. After that the suspension was cooled to the final crystallization temperature of 5°C and left at this temperature under stirring for 2 hours. The product was filtered off using vacuum filtration and washed with 43 mL of TBME.
Weight = 89.5 g
Molar yield = 68 %
Chromatographic purity = 96.5 area %
Enantiomeric purity = 50.03 area %
Content = 78.8 %

### Example 4a

### Optical resolution to obtain (R)-1-aminoindane N-acetyl-L-glutaminate

In a reactor 2.67 g of (R,S)-1-aminoindane N-acetyl-L-glutaminate and 80 mL of ethanol were added. Over the next 45 minutes the crystallization mixture was heated under stirring to 79°C. After reaching 79°C, the mixture was stirred at this temperature for 15 minutes, and the mixture was cooled within 45 minutes to 5°C. The mixture was left at this temperature overnight, under stirring. The precipitated product was isolated using vacuum filtration.
Weight = 1.21 g
Molar yield = 45 %
Chromatographic purity = 99.9 area %
Enantiomeric purity = 90.88 area %

### Example 4b

### Optical resolution to obtain (R)-1-aminoindane N-acetyl-L-glutaminate

In the reactor 70 g of (R, S)-1-aminoindane N-acetyl-L-glutaminate and 2100 mL of ethanol were added. Over the next 45 minutes the crystallization mixture was heated under stirring to temperature of 79°C. After reaching 79°C, the mixture was stirred at this temperature for 5 minutes and then was cooled within 45 minutes to a temperature of 40°C. At this temperature the mixture was seeded with 0.7 g of crystalline (R)-1-aminoindane N-acetyl-L-glutaminate. The mixture was stirred at this temperature for 20 minutes, and the suspension was cooled within 30 minutes to the final crystallization temperature of 5°C, and left at this temperature under stirring overnight. The product was isolated using vacuum filtration and washed with 30 mL of cold ethanol.
Weight = 29.9 g
Molar yield = 42.6%
Chromatographic purity = 99.97% area
Enantiomeric purity = 92.3% area
Content = 98.95%

### Example 5

### Recrystallization of (R)-1-aminoindane N-acetyl-L-glutaminate

Into the reactor 24 g of (R)-1-aminoindane N-acetyl-L-glutaminate and 1440 mL of ethanol were added. Over the next 45 minutes the crystallization mixture was heated under stirring to a temperature of 79°C. After reaching 79°C, 14.4 mL of water was added to the mixture and stirred at this temperature for 60 minutes. After that 240 mL of ethanol is added and the mixture was cooled within 45 minutes to a temperature 40°C. At this temperature the mixture was seeded with 0.24 g of crystalline (R)-1-aminoindane N-acetyl-L-glutaminate. The mixture was stirred at this temperature for 20 minutes, and then the suspension was cooled within 30 minutes to the final crystallization temperature of 5°C and left at this temperature under stirring overnight. The product was isolated using vacuum filtration and washed with 20 mL of cold ethanol.
Weight = 24.0 g
Molar yield = 95 %
Chromatographic purity = 100 % area %
Enantiomeric purity = 99.1 area %
Content = 95.9 %

### Example 6

### Salt neutralization

20 g of (S)-1-aminoindane N-acetyl-L-glutaminate, obtained from the filtrate of Example 4b, 200 mL 1 M NaOH and 200 mL ethyl acetate was charged to the reactor. The mixture was stirred for 0.5 h at room temperature, after that the phases were separated in a separating funnel. The water phase was washed with ethyl acetate; the organic phases were collected and concentrated under the reduced pressure to the constant weight to obtain 11.84 g of oily product (S)-1-aminoindane.
Molar yield: 98.8 %
Chromatographic purity: 99.83 %area
Enantiomeric purity: 99.36 %area

### Example 7

### Racemization of material comprising (S)-aminoindane enriched mixtures

6.53 g of (S)-1-aminoindane, 30 mL of toluene, 5 g of 1-indanone and 10.46 mL of triethylamine were charged into the reactor equipped with Dean-Stark apparatus. The reaction mixture was heated to reflux (T of 110°C to 115°C) and stirred at this temperature for 6 hours. During the reaction the conversion rate was monitored by means of HPLC. After the conversion was completed the reaction mixture was cooled to room temperature and 2.67 g of potassium t-butoxide was added to the reaction mixture. The reaction mixture was heated to reflux and stirred for 12 hours. After that the reaction mixture was cooled to room temperature, filtered and washed with 5 mL of methylene chloride. The solid was discarded; the filtrate was used in the process of hydrogenation which is in line with the process disclosed in Example 2. The final product of the reaction is racemic 1-aminoindane.

### Example 8

### Isolation of N-(2,3-dihydro-1H-inden-1-yl)-2,3-dihydro-1H-inden-1-imine by preparative chromatography

585.8 g of the reaction mixture from Example 2, which has been hydrogenated for 3.5 hours, were removed and N-(2,3-dihydro-1H-inden-1-yl)-2,3-dihydro-1H-inden-1-imine was isolated by preparative chromatography. The following chromatographic conditions were applied:
Column: X-Bridge C18 5um; 150X30mm
UV=210um
Mobile phase A: 0,01M (NH₃)H₂PO₄, pH=8,0
Mobile phase B: 90% Acetonitrile
Gradient: 0'=2%B; 10'=30%B; 29'-33'=100%; post run =2'
Flow rate: 42 ml/min

The starting sample comprised approximately 70 area% of N-(2,3-dihydro-1H-inden-1-yl)-2,3-dihydro-1H-inden-1-imine. Prior to the chromatographic separation the starting sample was filtered through a 45 µm filter disc. N-(2,3-dihydro-1H-inden-1-yl)-2,3-dihydro-1H-inden-1-imine was found to be very unstable as it starts to disintegrate already in the mobile phase at room temperature. Fractions were therefore collected in ice-cooled flasks and immediately frozen by means of liquid nitrogen and lyophilized. For the analysis and structure confirmation, acetonitrile was used as solvent for HPLC and LC/MS analyses, and deuterated chloroform for NMR analyses. 50 mg of N-(2,3-dihydro-1H-inden-1-yl)-2,3-dihydro-1H-inden-1-imine with chromatographic purity 85 % area were isolated.

¹H NMR spectrum of : ¹H NMR (CDCl₃)-δ: 7.90 (d, J=7.6 Hz, 1H), 7.38-7.43 (m, 1H), 7.35-7.38 (m, 1H), 7.26-7.31 (m, 2H), 7.14-7.23 (m, 2H), 7.08-7.13 (m, 1H), 5.12 (t, J=7.8 Hz, 1H), 3.14-3.19 (m, 2H), 3.08-3.13 (m, 1H), 2.96-3.01 (m, 1H), 2.90-2.96 (m, 2H), 2.44-2.56 (m, 1H), 2.10-2.23 (m, 1H)

### Example 9

### Racemization of material comprising (S)-aminoindane enriched mixtures (product RSF11-1K1601_6)

58,4 g of (S)-1-aminoindane, 268 mL of toluene, 44,7 g of 1-indanone and 93 mL of triethylamine were charged into the reactor equipped with Dean-Stark apparatus. The reaction mixture was heated to reflux (T of 108-109°C) and stirred at this temperature for 16 hours. During the reaction water was removed from the reaction mixture and the conversion rate was monitored by means of HPLC. After the conversion was completed the reaction mixture was cooled temperature of 100°C and 22.6 g of potassium hydroxide was added to the reaction mixture. The reaction mixture was heated to reflux and stirred for 4 hours. After that the reaction mixture was cooled to room temperature and filtered. The solid was discarded; the filtrate was evaporated under vacuum and 90.8g of the solid product was obtained. The product was dissolved in 1800 ml of methanol, subjected to nitrogen flow and used further in the process of hydrogenation in the presence of ammonia which was in line with the process disclosed in Example 2. The final product of the reaction is racemic 1-aminoindane.

### Example 10

### Preparation of racemic 1-aminoindane

### a) Condensation

150 g of enantiomerically enriched 1-aminoindane, comprising 62.8 area% of (S)-isomer and 32.1 area% of (R)-isomer, 660 mL of toluene, 111 g of 1-indanone and 226 mL of triethylamine were charged into the reactor equipped with Dean-Stark apparatus. The reaction mixture was heated to reflux (T of 108-109°C) and stirred at this temperature for 12 hours. During the reaction water was removed from the reaction mixture and the conversion rate was monitored by means of HPLC. After the conversion was completed the reaction mixture was cooled to room temperature and 55 g of potassium hydroxide was added to the reaction mixture. The reaction mixture was heated to reflux and stirred for 7 hours. After that the reaction mixture was cooled to room temperature and filtered. The solid was discarded; the filtrate was evaporated under vacuum and 225.62 g of the solid product was obtained. The product was dissolved in 3L of methanol, subjected to nitrogen flow and used further in the process of hydrogenation.

### b) Hydrogenation

Solution in methanol from step a) was cooled to 0°C and treated with 500g of gaseous ammonia. The obtained solution was charged in hydrogenation reactor and 26.6 g of Raney Ni, previously treated with methanol, was added to the reaction mixture. The mixture was hydrogenated by gaseous hydrogen under stirring at a constant pressure of 4 bars. The hydrogenation reaction was carried out at a temperature of 42 ± 2°C for 20 hours. After completion of the reaction, the reaction mixture was cooled to 5°C and the suspension was filtered to separate the catalyst. The filtrate was concentrated under reduced pressure at a temperature of less than 40 ° C to constant weight. 540 ml of an oily product were obtained (assay: 704 mg/ml, enantiomeric purity: 51 area% of (S)-isomer and 49 area% of (R)-isomer. The product was stored in an inert atmosphere at a temperature below 10°C and protected from light.

### Example 11

### Preparation of racemic 1-aminoindane

### a) Condensation

146 g of enantiomerically enriched 1-aminoindane, comprising 62.8 area% of (S)-isomer and 32.1 area% of (R)-isomer, 660 mL of toluene, 111 g of 1-indanone and 226 mL of triethylamine were charged into the reactor equipped with Dean-Stark apparatus. The reaction mixture was heated to reflux (T of 108-109°C) and stirred at this temperature for 23 hours. During the reaction water was removed from the reaction mixture and the conversion rate was monitored by means of HPLC. After the conversion was completed to the reaction mixture 55 g of potassium hydroxide was added. The reaction mixture heated under reflux and stirred for 23 hours. After that the reaction mixture was cooled to temperature of 30°C and filtered. The solid was discarded; the filtrate was evaporated under vacuum and 214.92 g of the solid product was obtained. The product was dissolved in 3 L ml of methanol, subjected to nitrogen flow and used further in the process of hydrogenation.

### b) Hydrogenation

Solution in methanol from step a) was cooled to 0°C and treated with 500g of gaseous ammonia. The obtained solution was charged in hydrogenation reactor and 26.6 g of Raney Ni, previously treated with methanol, was added to the reaction mixture. The mixture was hydrogenated under stirring at a constant pressure of hydrogen of 4 bar. The hydrogenation reaction was carried out at a temperature of 42°C for 20 hours. After completion of the reaction, the reaction mixture was cooled to 5°C and the suspension was filtered to separate the catalyst. The filtrate was concentrated under reduced pressure at a temperature of less than 40 ° C to constant weight. 240 ml of an oily product were obtained (assay: 631 mg/ml, enantiomeric purity: 51.7 area% of (S)-isomer and 48.2 area% of (R)-isomer). The product was stored in an inert atmosphere at a temperature below 10°C and protected from light.

### Example 12

### Preparation of rasagiline

To a reactor 8.14 g of propargyl benzenesulphonate, 75.71 g of (R)-1-aminoindane hydrochloride and 57 ml of toluene were added. The mixture was heated to 30°C, and then 18 ml of 15% NaOH were slowly added, the mixture was further stirred at the same temperature for 29 hours. After completion of the reaction 38.5 ml of water and 16 ml of toluene were added to the reaction mixture and stirred for 15 minutes, then the stirring was stopped and the phases were separated. The organic phase was washed with 38.5 ml of water and stirred for 15 minutes, then the phases were separated. The upper organic phase was washed with 38.5 ml of 10% NaOH and stirred for 15 minutes, then the phases were separated. The organic phase was mixed with 23 ml of water and the pH was adjusted to 3.2 by addition of 12 ml of 10% solution of H₂SO₄. The phases were separated and the organic phase was discarded. To the water phase 38.5 ml of toluene were added and the pH was adjusted to 7.3 by adding 9 ml of 10% NaOH. The phases were separated and the water phase was extracted twice by addition of 38.5 ml of toluene and adjusting pH to 7.3 by addition of 10% solution of NaOH. Organic phases were collected and the solvent of the organic phase was evaporated under vacuum by heating and stirring. After the evaporation 4.87 g of an oily product with an assay of 74.9% was obtained.
Yield: 65.8%,
Enantiomeric purity: 99.96 area %

## Claims

1. A process for the racemization of an enantiomerically enriched (S)-1-aminoindane mixture comprising the following steps:
(a) providing a material comprising (S)-1-aminoindane enriched mixtures;
(b) mixing the material with 1-indanone and at least one organic solvent in the presence of an aprotic base to form an optically active compound of formula II, 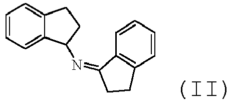
(c) racemization of the Schiff base of formula II by treatment with a base,
(d) hydrogenation of the racemic compound of formula II, to obtain racemic or nearly racemic 1-aminoindane.

2. A process according to claim 1 wherein the material comprises (S)-1-aminoindane in the form of an acid addition salt of (S)-1-aminoindane.

3. A process according to claim 2 wherein the acid addition salt of (S)-1-aminoindane is neutralized to form (S)-1-aminoindane prior to the racemization process.

4. A process according to any one of claims 1 to 3, wherein the organic solvent used in step (b) is a non-polar solvent, an alcohol or a mixture thereof, wherein the non-polar solvent is preferably selected from toluene, xylene, tetrahydrofuran, cyclohexane, hexane, 1,4-dioxane and mixtures thereof, and wherein the alcohol is preferably selected from methanol, ethanol, propanol, isopropanol, butanol, sec-butanol and mixtures thereof.

5. A process according to any one of claims 1 to 4, wherein the aprotic base used in step (b) is selected from the group consisting of primary alkylamines, secondary alkylamines, tertiary alkylamines, heterocyclic amines or an alkyl substituted derivative thereof, inorganic bases, metal hydroxides, alkali alcoholates, alkali metal alcoholates and mixtures thereof.

6. A process according to any one of claims 1 to 5, wherein the molar ratio of 1-indanone used in step (b) to the amount of 1-aminoindane in the (S)-1-aminoindane enriched mixture ranges from 0.5:1 to 1.5:1.

7. A process according to claim 6, wherein the molar ratio of 1-indanone used in step (b) to the amount of 1-aminoindane in the (S)-1-aminoindane enriched mixture ranges from 0.6:1 to 1.2:1.

8. A process according to claim 7, wherein the molar ratio of 1-indanone used in step (b) to the amount of 1-aminoindane in the (S)-1-aminoindane enriched mixture ranges from 0.7:1 to 0.8:1.

9. A process according to any one of claims 1 to 8, wherein in step (b) the reaction mixture is heated to a temperature of 40°C to the boiling temperature of the solvent used in step (b) .

10. A process according to any one of claims 1 to 9, wherein the base used in step (c) is selected from alkali metal alcoholates, tertiary amines, alkali hydroxides or mixtures thereof.

11. A process according to any one of claims 1 to 10, wherein in step (c) the reaction mixture is heated to a temperature of 40°C to the boiling temperature of the reaction mixture.

12. A process according to any one of claims 1 to 11, wherein the hydrogenation in step (d) is performed in the presence of ammonia.

13. Process for preparing rasagiline or a pharmaceutically acceptable salt thereof, comprising
(a) preparing racemic 1-aminoindane by a process according to any one of claims 1 to 12,
(b) preparing optically pure (R)-1-aminoindane from the racemic 1-aminoindane, and
(c) converting the optically pure (R)-1-aminoindane into rasagiline or a pharmaceutically acceptable salt thereof.

14. A process for the preparation of rasagiline according to claim 13, wherein propargyl chloride and (R)-1-aminoindane from a racemic, or nearly racemic mixture of (R)-1-aminoindane and (S)-1-aminoindane, are reacted, and wherein the remaining unreacted (S)-1-aminoindane is racemized via the process according to any of claims 1 to 12, and returned to react with propargyl chloride.

## Patentansprüche

1. Verfahren zur Racemisierung einer enantiomerenangereicherten (S)-1-Aminoindan-Mischung, bei dem:
a) ein Material bereitgestellt wird, das mit (S)-1-Aminoindan angereicherte Mischungen enthält,
b) das Material mit 1-Indanon und mindestens einem organischen Lösungsmittel in der Gegenwart einer aprotischen Base gemischt wird, um eine optisch aktive Verbindung der Formel II zu bilden, 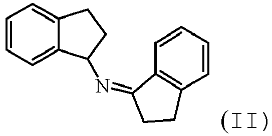
c) die Schiff'sche Base der Formel II durch Behandlung mit einer Base racemisiert wird,
d) die racemische Verbindung der Formel II hydriert wird, um racemisches oder nahezu racemisches 1-Aminoindan zu erhalten.

2. Verfahren nach Anspruch 1, bei dem das Material (S)-1-Aminoindan in der Form eines Säureadditionssalzes von (S)-1-Aminoindan enthält.

3. Verfahren nach Anspruch 2, bei dem das Säureadditionssalz von (S)-1-Aminoindan vor dem Racemisierungsverfahren unter Bildung von (S)-1-Aminoindan neutralisiert wird.

4. Verfahren nach einem der Ansprüche 1 bis 3, bei dem das in Schritt (b) verwendete organische Lösungsmittel ein unpolares Lösungsmittel, ein Alkohol oder eine Mischung davon ist, wobei das unpolare Lösungsmittel vorzugsweise aus Toluol, Xylol, Tetrahydrofuran, Cyclohexan, Hexan, 1,4-Dioxan und Mischungen davon ausgewählt ist und wobei der Alkohol vorzugsweise aus Methanol, Ethanol, Propanol, Isopropanol, Butanol, sec-Butanol und Mischungen davon ausgewählt ist.

5. Verfahren nach einem der Ansprüche 1 bis 4, bei dem die in Schritt b) verwendete aprotische Base ausgewählt ist aus der Gruppe bestehend aus primären Alkylaminen, sekundären Alkylaminen, tertiären Alkylaminen, heterocylischen Aminen oder einem alkylsubstituierten Derivat davon, anorganischen Basen, Metallhydroxiden, Alkalialkoholaten, Alkalimetallalkoholaten und Mischungen davon.

6. Verfahren nach einem der Ansprüche 1 bis 5, bei dem das molare Verhältnis von in Schritt b) verwendetem 1-Indanon zu der Menge von 1-Aminoindan in der mit (S)-1-Aminoindan angereicherten Mischung von 0,5:1 bis 1,5:1 reicht.

7. Verfahren nach Anspruch 6, bei dem das molare Verhältnis von in Schritt b) verwendetem 1-Indanon zu der Menge von 1-Aminoindan in der mit (S)-1-Aminoindan angereicherten Mischung von 0,6:1 bis 1,2:1 reicht.

8. Verfahren nach Anspruch 7, bei dem das molare Verhältnis von in Schritt b) verwendetem 1-Indanon zu der Menge von 1-Aminoindan in der mit (S)-1-Aminoindan angereicherten Mischung von 0,7:1 bis 0,8:1 reicht.

9. Verfahren nach einem der Ansprüche 1 bis 8, bei dem in Schritt b) die Reaktionsmischung bis auf eine Temperatur von 40°C bis zur Siedetemperatur des in Schritt b) verwendeten Lösungsmittels erhitzt wird.

10. Verfahren nach einem der Ansprüche 1 bis 9, bei dem die in Schritt c) verwendete Base aus Alkalimetallalkoholaten, tertiären Aminen, Alkalihydroxiden oder Mischungen davon ausgewählt ist.

11. Verfahren nach einem der Ansprüche 1 bis 10, bei dem in Schritt c) die Reaktionsmischung auf eine Temperatur von 40°C bis zur Siedetemperatur der Reaktionsmischung erhitzt wird.

12. Verfahren nach einem der Ansprüche 1 bis 11, bei dem die Hydrierung in Schritt d) in der Gegenwart von Ammoniak durchgeführt wird.

13. Verfahren zur Herstellung von Rasagilin oder eines pharmazeutisch annehmbaren Salzes davon, bei dem
a) racemisches 1-Aminoindan durch ein Verfahren gemäß einem der Ansprüche 1 bis 12 hergestellt wird,
b) optisch reines (R)-1-Aminoindan aus dem racemischen 1-Aminoindan hergestellt wird und
c) das optisch reine (R)-1-Aminoindan in Rasagilin oder ein pharmazeutisch annehmbares Salz davon umgewandelt wird.

14. Verfahren zur Herstellung von Rasagilin nach Anspruch 13, bei dem Propargylchlorid und (R)-1-Aminoindan aus einer racemischen oder nahezu racemischen Mischung von (R)-1-Aminoindan und (S)-1-Aminoindan umgesetzt werden und bei dem das verbleibende nicht umgesetzte (S)-1-Aminoindan durch das Verfahren gemäß einem der Ansprüche 1 bis 12 racemisiert wird und zurückgeführt wird, um mit Propargylchlorid zu reagieren.

## Revendications

1. Procédé de racémisation d'un mélange d'énantiomères de 1-amino-indane enrichi en énantiomère S, comportant les étapes suivantes :
a) prendre un matériau comprenant un mélange enrichi en (S)-1-amino-indane,
b) mélanger ce matériau avec de l'indan-1-one et au moins un solvant organique, en présence d'une base aprotique, pour qu'il se forme un composé optiquement actif de formule II : 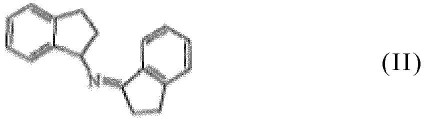
c) provoquer la racémisation de cette base de Schiff de formule II, en la traitant avec une base,
d) et opérer une hydrogénation du composé racémique de formule II, pour obtenir du 1-amino-indane racémique ou presque racémique.

2. Procédé conforme à la revendication 1, dans lequel le matériau comprend du (S)-1-amino-indane à l'état de sel d'addition d'acide de (S)-1-amino-indane.

3. Procédé conforme à la revendication 2, dans lequel le sel d'addition d'acide de (S)-1-amino-indane est neutralisé avant le procédé de racémisation, pour donner du (S)-1-amino-indane.

4. Procédé conforme à l'une des revendications 1 à 3, dans lequel le solvant organique utilisé dans l'étape (b) est un solvant non-polaire, un alcool ou un mélange de tels solvants, étant entendu que le solvant non-polaire est de préférence choisi parmi les toluène, xylène, tétrahydrofurane, cyclohexane, 1,4-dioxane et leurs mélanges, et que l'alcool est de préférence choisi parmi les méthanol, éthanol, propanol, isopropanol, butanol, *sec*-butanol et leurs mélanges.

5. Procédé conforme à l'une des revendications 1 à 4, dans lequel la base aprotique utilisée dans l'étape (b) est choisie dans l'ensemble formé par les alkyl-amines primaires, alkyl-amines secondaires, alkyl-amines tertiaires, amines hétérocycliques ou leurs dérivés à substituant(s) alkyle, bases inorganiques, hydroxydes de métaux, alcoolates d'alcali et alcoolates de métal alcalin, ainsi que les mélanges de telles bases.

6. Procédé conforme à l'une des revendications 1 à 5, dans lequel le rapport molaire de l'indan-1-one utilisée dans l'étape (b) au 1-amino-indane présent dans le mélange enrichi en (S)-1-amino-indane vaut de 0,5/1 à 1,5/1.

7. Procédé conforme à la revendication 6, dans lequel le rapport molaire de l'indan-1-one utilisée dans l'étape (b) au 1-amino-indane présent dans le mélange enrichi en (S)-1-amino-indane vaut de 0,6/1 à 1,2/1.

8. Procédé conforme à la revendication 7, dans lequel le rapport molaire de l'indan-1-one utilisée dans l'étape (b) au 1-amino-indane présent dans le mélange enrichi en (S)-1-amino-indane vaut de 0,7/1 à 0,8/1.

9. Procédé conforme à l'une des revendications 1 à 8, dans lequel, dans l'étape (b), le mélange réactionnel est chauffé à une température qui vaut de 40°C au point d'ébullition du solvant utilisé dans l'étape (b).

10. Procédé conforme à l'une des revendications 1 à 9, dans lequel la base utilisée dans l'étape (c) est choisie parmi les alcoolates de métal alcalin, les amines tertiaires, les hydroxydes de métal alcalin et les mélanges de telles bases.

11. Procédé conforme à l'une des revendications 1 à 10, dans lequel, dans l'étape (c), le mélange réactionnel est chauffé à une température qui vaut de 40°C au point d'ébullition du mélange réactionnel.

12. Procédé conforme à l'une des revendications 1 à 11, dans lequel, dans l'étape (d), l'hydrogénation est effectuée en présence d'ammoniac

13. Procédé de préparation de rasagiline ou d'un sel pharmacologiquement admissible de rasagiline, comportant les étapes suivantes :
a) préparer du 1-amino-indane racémique, par un procédé conforme à l'une des revendications 1 à 12,
b) préparer du (R)-1-amino-indane optiquement pur, à partir de ce 1-amino-indane racémique,
c) et convertir ce (R)-1-amino-indane optiquement pur en rasagiline ou en sel pharmacologiquement admissible de rasagiline.

14. Procédé de préparation de rasagiline, conforme à la revendication 13, dans lequel on fait réagir du chlorure de propargyle et du (R)-1-amino-indane issu d'un mélange racémique, ou presque racémique, de (R)-1-amino-indane et de (S)-1-amino-indane, et dans lequel le (S)-1-amino-indane restant, qui n'a pas réagi, est racémisé selon un procédé conforme à l'une des revendications 1 à 12, puis renvoyé réagir avec du chlorure de propargyle.
